# EUROPEAN PATENT APPLICATION

(11) **EP 4 679 375 A1**
(43) Date of publication of application: **14.01.2026**
(21) Application number: 25186803.0
(22) Date of filing: 01.07.2025
(51) Int. Cl.: G06T 11/00

(54) **IMAGE GENERATION APPARATUS, IMAGE GENERATION METHOD, AND PROGRAM**

(30) Priority: 12.07.2024 JP 2024112890
(71) Applicant: FUJIFILM Corporation, Tokyo Tokyo 106-8620 (JP)
(72) Inventor: INOUE, Tomoki, Kanagawa (JP); FUKUDA, Wataru, Kanagawa (JP)
(74) Representative: Dehns Germany Partnerschaft mbB

(57) **Abstract**

Provided are an image generation apparatus (16), an image generation method, and a program capable of improving generation accuracy of a pseudo two-dimensional image (102; 103), as compared with a case of using an image generation model that has been trained using a combination of a plurality of projection images or a plurality of tomographic images and a normal two-dimensional image captured separately from tomosynthesis imaging for obtaining the plurality of projection images or the plurality of tomographic images.

An image processing apparatus as an image generation apparatus includes an image generation model (67) that has been trained in advance using a plurality of combinations of a plurality of projection images or a plurality of tomographic images (100; 101) and a normal two-dimensional image (111) captured by irradiating, with radiation (R), a breast (U) in a state of being compressed by a compression member (30) during tomosynthesis imaging for obtaining the plurality of projection images or the plurality of tomographic images.

## Description

### BACKGROUND OF THE INVENTION

### 1. Field of the Invention

The present disclosure relates to an image generation apparatus, an image generation method, and a program.

### 2. Description of the Related Art

JP2023-51400A discloses a training device for an image generation model that generates a pseudo two-dimensional image in which a shape of a lesion of a breast is accurately reproduced.

The training device is a device for training an image generation model that generates a pseudo two-dimensional image from a series of a plurality of projection images obtained by tomosynthesis imaging for a breast or a plurality of tomographic images obtained from the series of the plurality of projection images, the training device comprising: at least one processor, in which the processor acquires a normal two-dimensional image captured by irradiating the breast with radiation, detects a first region of interest including calcification of the breast and a second region of interest including a lesion other than the calcification based on any of a composite two-dimensional image obtained by combining at least some of the series of the plurality of projection images or the plurality of tomographic images, the tomographic image, or the normal two-dimensional image, and trains the image generation model by updating a weight of a network of the image generation model to reduce a loss based on a loss of the pseudo two-dimensional image output by the image generation model in which a weight of the first region of interest is set to be the greatest and a weight of the second region of interest is set to be equal to or greater than a weight of a region other than the first region of interest and the second region of interest, and the normal two-dimensional image, the composite two-dimensional image, or both of the normal two-dimensional image and the composite two-dimensional image.

### SUMMARY OF THE INVENTION

It is preferable that a combination of the image obtained by the tomosynthesis imaging and the normal two-dimensional image, which is used for the training of the image generation model, is a combination of the same breast images, which are obtained in the same state in which the breast is in a compression state by a compression member. In a case in which the combination of the images used for training is different breast images or images in the different compression states, a breast state such as a size, a thickness, a shape, and a position is different between the breast shown by the image obtained by the tomosynthesis imaging and the breast shown by the normal two-dimensional image. Therefore, training with such a combination of images leads to decreased generation accuracy of the pseudo two-dimensional image using the image generation model.

However, in the technology disclosed in JP2023-51400A, the combination of the image obtained by the tomosynthesis imaging and the normal two-dimensional image, which is used for training, is the combination of the images obtained by imaging the same breast, but includes the combination of the images that are not obtained in the same compression state. Therefore, the technology disclosed in JP2023-51400A has a problem in that the generation accuracy of the pseudo two-dimensional image using a trained image generation model is not always high.

The present disclosure has been made in view of the above-described circumstances, and an object of the present disclosure is to provide an image generation apparatus, an image generation method, and a program capable of improving generation accuracy of a pseudo two-dimensional image, as compared with a case of using an image generation model that has been trained using a combination of a plurality of projection images or a plurality of tomographic images and a normal two-dimensional image captured separately from tomosynthesis imaging for obtaining the plurality of projection images or the plurality of tomographic images.

In order to achieve the above-described object, a first aspect of the present disclosure provides an image generation apparatus that generates a pseudo two-dimensional image from a series of a plurality of projection images obtained by tomosynthesis imaging in a state in which a breast is compressed by a compression member or a plurality of tomographic images obtained from the series of the plurality of projection images, the image generation apparatus comprising: at least one image generation model, in which the image generation model has been trained in advance using a plurality of combinations of the plurality of projection images or the plurality of tomographic images and a normal two-dimensional image captured by irradiating, with radiation, the breast in a state of being compressed by the compression member during the tomosynthesis imaging for obtaining the plurality of projection images or the plurality of tomographic images.

A second aspect of the present disclosure provides the image generation apparatus according to the first aspect, further comprising: a processor, in which the processor is configured to: acquire the plurality of combinations of the plurality of projection images or the plurality of tomographic images and the normal two-dimensional image, for each predetermined category; and train a plurality of the image generation models by individually training the image generation model for each category.

A third aspect of the present disclosure provides the image generation apparatus according to the second aspect, in which the predetermined category is a category related to a person under examination.

A fourth aspect of the present disclosure provides the image generation apparatus according to the third aspect, in which the category related to the person under examination includes at least one category of a height, a weight, a body mass index (BMI), an age, mammary gland information, or a thickness of a breast of the person under examination.

A fifth aspect of the present disclosure provides the image generation apparatus according to the second aspect, in which the predetermined category is a category related to a setting content during imaging.

A sixth aspect of the present disclosure provides the image generation apparatus according to the fifth aspect, in which the category related to the setting content during the imaging includes at least one category of an irradiation angle of the radiation, a resolution of a captured image, or a dose mode of the radiation during the tomosynthesis imaging.

A seventh aspect of the present disclosure provides the image generation apparatus according to the sixth aspect, in which the category of the dose mode of the radiation is a category for each combination of a first dose mode during the tomosynthesis imaging and a second dose mode that is a dose mode in a case of capturing the normal two-dimensional image and that has a dose equal to or greater than a dose in the first dose mode.

An eighth aspect of the present disclosure provides the image generation apparatus according to the second aspect, in which the predetermined category is a category related to a type of an image processing technique for at least one of the plurality of projection images or the plurality of tomographic images or the normal two-dimensional image.

A ninth aspect of the present disclosure provides the image generation apparatus according to the eighth aspect, in which the category related to the type of the image processing technique is a category for each manufacturer of an imaging apparatus that performs the tomosynthesis imaging.

A tenth aspect of the present disclosure provides the image generation apparatus according to any one of the second to ninth aspects, in which the processor is configured to: in a case in which an image quality of at least one image in the plurality of combinations of the plurality of projection images or the plurality of tomographic images and the normal two-dimensional image is lower than a predetermined level, prohibit use of the images of the combination for the training.

An eleventh aspect of the present disclosure provides the image generation apparatus according to the second aspect, in which the processor is configured to: in a case in which the plurality of tomographic images are used for the training, correct the plurality of tomographic images to tomographic images in a case of virtually providing a tube at a position of the tube that emits the radiation in a case of capturing a corresponding normal two-dimensional image.

A twelfth aspect of the present disclosure provides the image generation apparatus according to the second aspect, in which the processor is configured to: in a case in which a set of images, which is the combination of the plurality of projection images or the plurality of tomographic images and the normal two-dimensional image, is obtained by set imaging in which the tomosynthesis imaging and the capturing of the normal two-dimensional image are performed in the same compression state by the compression member, use the set of images as training data for the image generation model.

A thirteenth aspect of the present disclosure provides the image generation apparatus according to the twelfth aspect, in which the processor is configured to: accumulate the set of images and use the set of images for the training of the image generation model.

A fourteenth aspect of the present disclosure provides the image generation apparatus according to the twelfth or thirteenth aspect, in which the processor is configured to: sequentially update the image generation model using the set of images.

A fifteenth aspect of the present disclosure provides the image generation apparatus according to the second aspect, in which the processor is configured to: acquire the series of the plurality of projection images obtained by the tomosynthesis imaging in a state in which the breast is compressed by the compression member or the plurality of tomographic images obtained from the series of the plurality of projection images, as images for generation of the pseudo two-dimensional image; and input the acquired images for generation to the image generation model to generate the pseudo two-dimensional image corresponding to the images for generation.

A sixteenth aspect of the present disclosure provides the image generation apparatus according to the fifteenth aspect, in which the processor is configured to: in a case in which the plurality of image generation models that have been individually trained for each predetermined category are provided as the image generation model, generate the pseudo two-dimensional image by selectively using the image generation model of the category corresponding to the pseudo two-dimensional image to be generated among the plurality of image generation models.

A seventeenth aspect of the present disclosure provides the image generation apparatus according to the first or second aspect, in which the image generation model is provided in an imaging apparatus that performs only the tomosynthesis imaging or an imaging apparatus that separately performs the tomosynthesis imaging and mammography imaging.

In addition, in order to achieve the above-described object, an eighteenth aspect of the present disclosure provides an image generation method executed by a computer, the image generation method comprising: generating, via an image generation model, a pseudo two-dimensional image from a series of a plurality of projection images obtained by tomosynthesis imaging in a state in which a breast is compressed by a compression member or a plurality of tomographic images obtained from the series of the plurality of projection images, in which the image generation model has been trained in advance using a plurality of combinations of the plurality of projection images or the plurality of tomographic images and a normal two-dimensional image captured by irradiating, with radiation, the breast in a state of being compressed by the compression member during the tomosynthesis imaging for obtaining the plurality of projection images or the plurality of tomographic images.

In addition, in order to achieve the above-described object, a nineteenth aspect of the present disclosure provides a program causing a computer to execute a process comprising: generating, via an image generation model, a pseudo two-dimensional image from a series of a plurality of projection images obtained by tomosynthesis imaging in a state in which a breast is compressed by a compression member or a plurality of tomographic images obtained from the series of the plurality of projection images, in which the image generation model has been trained in advance using a plurality of combinations of the plurality of projection images or the plurality of tomographic images and a normal two-dimensional image captured by irradiating, with radiation, the breast in a state of being compressed by the compression member during the tomosynthesis imaging for obtaining the plurality of projection images or the plurality of tomographic images.

According to the present disclosure, it is possible to improve the generation accuracy of the pseudo two-dimensional image, as compared with a case of using the image generation model that has been trained using the combination of the plurality of projection images or the plurality of tomographic images and the normal two-dimensional image captured separately from the tomosynthesis imaging for obtaining the plurality of projection images or the plurality of tomographic images.

### BRIEF DESCRIPTION OF THE DRAWINGS

Fig. 1 is a configuration diagram schematically showing an example of an overall configuration of a radiographic imaging system according to an embodiment.
Fig. 2 is a diagram showing an example of tomosynthesis imaging.
Fig. 3 is a diagram showing an example of an image generation model.
Fig. 4 is a diagram showing an example of an intermediate layer of the image generation model shown in Fig. 3.
Fig. 5 is a block diagram showing an example of a configuration of an image processing apparatus according to the embodiment.
Fig. 6 is a schematic diagram showing an outline of a flow of training of each image generation model of an image generation model group in the image processing apparatus according to the embodiment.
Fig. 7 is a functional block diagram showing an example of a configuration related to a function of training the image generation model in the image processing apparatus of the embodiment.
Fig. 8 is a schematic diagram showing an example of a configuration of a model selection information database according to the embodiment.
Fig. 9 is a flowchart showing an example of a flow of training processing performed by the image processing apparatus according to the embodiment.
Fig. 10 is a schematic diagram showing an outline of a flow of generating a pseudo two-dimensional image using the image generation model in the image processing apparatus according to the embodiment.
Fig. 11 is a functional block diagram showing an example of a configuration related to a function of generating the pseudo two-dimensional image in the image processing apparatus of the embodiment.
Fig. 12 is a flowchart showing an example of a flow of image generation processing performed by the image processing apparatus of the embodiment.
Fig. 13 is a schematic diagram showing an example of another configuration of the model selection information database according to the embodiment.
Fig. 14 is a schematic diagram showing an example of a configuration of the model selection information database according to the embodiment.
Fig. 15 is a schematic diagram showing an example of another configuration of the model selection information database according to the embodiment.
Fig. 16 is a schematic diagram showing an example of a configuration of the model selection information database according to the embodiment.
Fig. 17 is a schematic diagram showing an example of another configuration of the model selection information database according to the embodiment.
Fig. 18A is a diagram showing examples of an irradiation position of radiation and a normal two-dimensional image in a case in which normal imaging according to the embodiment is performed.
Fig. 18B is a diagram showing examples of the irradiation position of the radiation and a projection image in a case in which a reference position is shifted from a reference position during the normal imaging in a case in which the tomosynthesis imaging according to the embodiment is performed.

### DESCRIPTION OF THE PREFERRED EMBODIMENTS

Hereinafter, embodiments of the present disclosure will be described in detail with reference to the drawings. The present embodiment does not limit the technology of the present disclosure.

### First Embodiment

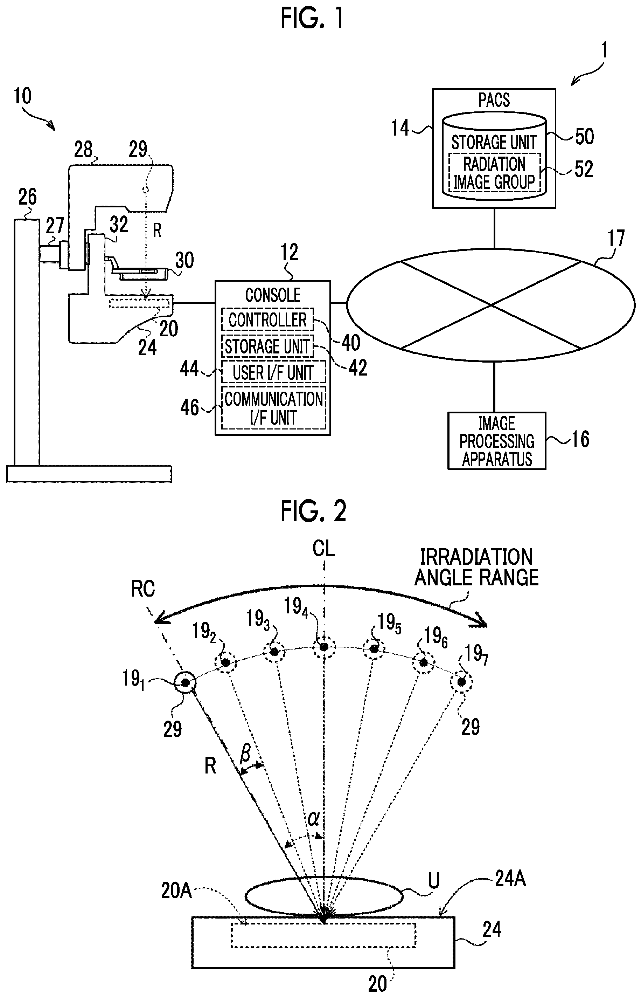

First, an example of an overall configuration of a radiographic imaging system according to the present embodiment to which the technology of the present disclosure is applied will be described. Fig. 1 is a configuration diagram showing an example of an overall configuration of a radiographic imaging system 1 according to the present embodiment. As shown in Fig. 1, the radiographic imaging system 1 according to the present embodiment comprises a mammography apparatus 10, a console 12, a picture archiving and communication systems (PACS) 14, and an image processing apparatus 16. The console 12, the PACS 14, and the image processing apparatus 16 are connected via a network 17 by wired communication or wireless communication.

First, the mammography apparatus 10 according to the present embodiment will be described. In Fig. 1, a side view showing an example of an appearance of the mammography apparatus 10 according to the present embodiment is shown. It should be noted that Fig. 1 shows an example of the appearance in a case in which the mammography apparatus 10 is viewed from a left side of a person under examination.

The mammography apparatus 10 according to the present embodiment is an apparatus that is operated under the control of the console 12, and is configured to capture, using a breast of the person under examination as a subject, a radiation image of a breast by irradiating the breast with radiation R (for example, X-rays) emitted from a radiation source 29. Further, the mammography apparatus 10 according to the present embodiment has a function of performing normal imaging for capturing images by arranging the radiation source 29 at an irradiation position along a normal direction to a detection surface 20A (see also Fig. 2) of a radiation detector 20 and so-called tomosynthesis imaging (which will be described in detail below) for capturing images by moving the radiation source 29 to each of a plurality of irradiation positions.

As shown in Fig. 1, the mammography apparatus 10 comprises an imaging table 24, a base 26, an arm portion 28, and a compression unit 32.

A radiation detector 20 is disposed inside the imaging table 24. As shown in Fig. 2, in the mammography apparatus 10 according to the present embodiment, in a case in which the imaging is performed, a breast U of the person under examination is positioned on an imaging surface 24A of the imaging table 24 by a user.

The radiation detector 20 detects the radiation R that has been transmitted through the breast U as the subject. Specifically, the radiation detector 20 detects the radiation R that enters the breast U of the person under examination and the imaging table 24 and that reaches the detection surface 20A of the radiation detector 20, generates a radiation image based on the detected radiation R, and outputs image data representing the generated radiation image. Hereinafter, the series of operations of irradiating the breast with the radiation R emitted from the radiation source 29 to generate the radiation image via the radiation detector 20 may be referred to as "imaging". A type of the radiation detector 20 according to the present embodiment is not particularly limited, and for example, the radiation detector 20 may be an indirect conversion type radiation detector that converts the radiation R into light and converts the converted light into charge, or may be a direct conversion type radiation detector that directly converts the radiation R into charge.

The compression plate 30 as a compression member of the present disclosure used to compress the breast in a case of performing imaging is attached to the compression unit 32 provided on the imaging table 24 and is moved in a direction approaching or departing from the imaging table 24 (hereinafter, referred to as an "up-down direction") by a compression plate drive unit (not shown) provided in the compression unit 32. The compression plate 30 is moved in the up-down direction to compress the breast of the person under examination between the imaging table 24 and the compression plate 30.

The arm portion 28 can rotate relative to the base 26 via a shaft portion 27. The shaft portion 27 is fixed to the base 26, and the shaft portion 27 and the arm portion 28 rotate as one body. Gears are provided in each of the shaft portion 27 and the compression unit 32 of the imaging table 24, and the gears are switched between an engaged state and a non-engaged state, so that a state in which the compression unit 32 of the imaging table 24 and the shaft portion 27 are connected to each other and are rotated integrally and a state in which the shaft portion 27 is separated from the imaging table 24 and idles can be switched. The elements for switching between transmission and non-transmission of power of the shaft portion 27 are not limited to the gears, and various mechanical elements can be used. The arm portion 28 and the imaging table 24 can be separately rotated relative to the base 26 with the shaft portion 27 as a rotation axis.

In a case in which the tomosynthesis imaging is performed in the mammography apparatus 10, the radiation source 29 is sequentially moved to each of the plurality of irradiation positions having different irradiation angles in accordance with the rotation of the arm portion 28. The radiation source 29 has a radiation tube (not shown) as a tube of the present disclosure that generates the radiation R, and the radiation tube is moved to each of the plurality of irradiation positions in accordance with the movement of the radiation source 29.

Fig. 2 is a diagram showing an example of the tomosynthesis imaging. It should be noted that, in Fig. 2, the compression plate 30 is not shown. In the present embodiment, as shown in Fig. 2, the radiation source 29 is moved to irradiation positions 19t (t = 1, 2, ▪▪▪; the maximum value is 7 in Fig. 2) having different irradiation angles at an interval of a predetermined angle β, that is, positions at which the irradiation angle of the radiation R with respect to the detection surface 20A of the radiation detector 20 are different from each other. At each irradiation position 19ₜ, the breast U is irradiated with the radiation R emitted from the radiation source 29 in accordance with an instruction of the console 12, and the radiation image is captured by the radiation detector 20. In the radiographic imaging system 1, in a case in which the tomosynthesis imaging is performed by moving the radiation source 29 to each irradiation position 19ₜ to capture the radiation image at each irradiation position 19t, seven radiation images are obtained in the example of Fig. 2. Hereinafter, during the tomosynthesis imaging, in a case in which the radiation image captured at each irradiation position 19 is described separately from other radiation images, the radiation image is referred to as a "projection image", and a plurality of projection images captured in one tomosynthesis imaging are referred to as a "series of a plurality of projection images". Further, in a case of referring to the radiation images collectively, regardless of the type, such as the projection images, a tomographic images and normal two-dimensional images which are described later, the term "radiation image" is simply used. In addition, in the following description, for the images corresponding to the irradiation positions 19t, such as the projection images captured at the irradiation positions 19t, the reference symbol "t" representing the irradiation position 19ₜ is added to the reference numeral representing each image.

It should be noted that, as shown in Fig. 2, the irradiation angle of the radiation R refers to an angle α formed between a normal line CL of the detection surface 20A of the radiation detector 20 and a radiation axis RC. The radiation axis RC refers to an axis that connects a focus of the radiation source 29 at each irradiation position 19 and a preset position, such as a center of the detection surface 20A. Further, here, it is assumed that the detection surface 20A of the radiation detector 20 is substantially parallel to the imaging surface 24A.

Meanwhile, in a case in which the mammography apparatus 10 performs the normal imaging, the radiation source 29 remains at the irradiation position 19ₜ (the irradiation position 19ₜ along the normal direction, the irradiation position 19₄ in Fig. 2) at which the irradiation angle α is 0 degrees. The radiation R is emitted from the radiation source 29 in accordance with the instruction of the console 12, and the radiation image is captured by the radiation detector 20. In the present embodiment, the radiation image captured during the normal imaging is referred to as a "normal two-dimensional image" in a case in which the radiation image is described as being distinguished from other radiation images.

The mammography apparatus 10 and the console 12 are connected by wired communication or wireless communication. The radiation image captured by the radiation detector 20 in the mammography apparatus 10 is output to the console 12 by wired communication or wireless communication via a communication interface (I/F) unit (not shown).

As shown in Fig. 1, the console 12 according to the present embodiment comprises a controller 40, a storage unit 42, a user I/F unit 44, and a communication I/F unit 46.

As described above, the controller 40 of the console 12 has a function of controlling the capturing of the radiation image of the breast via the mammography apparatus 10. Examples of the controller 40 include a computer system comprising a central processing unit (CPU), a read-only memory (ROM), and a random-access memory (RAM).

The storage unit 42 has a function of storing, for example, information on the capturing of the radiation image, or the radiation image acquired from the mammography apparatus 10. The storage unit 42 is a non-volatile storage unit and is, for example, a hard disk drive (HDD) or a solid state drive (SSD).

The user I/F unit 44 includes input devices, such as various buttons and switches operated by the user such as a technician, regarding the capturing of the radiation image, and display devices, such as a lamp and a display, for displaying information on the imaging or the radiation image.

The communication I/F unit 46 performs communication of various types of data, such as information on the capturing of the radiation image or the radiation image obtained by the imaging, to and from the mammography apparatus 10 by wired communication or wireless communication. In addition, the communication I/F unit 46 performs communication of various types of data, such as the radiation image, with the PACS 14 and the image processing apparatus 16 via the network 17 by wired communication or wireless communication.

In addition, as shown in Fig. 1, the PACS 14 according to the present embodiment comprises a storage unit 50 that stores a radiation image group 52 and a communication I/F unit (not shown). The radiation image group 52 includes radiation images that are captured by the mammography apparatus 10 and that are acquired from the console 12 via a communication I/F unit (not shown) and the like.

The image processing apparatus 16 is used by a doctor or the like (hereinafter, simply referred to as "doctor") to interpret the radiation image. The image processing apparatus 16 according to the present embodiment has a function of generating a pseudo two-dimensional image corresponding to the normal two-dimensional image from a plurality of tomographic images using an image generation model. It should be noted that the image processing apparatus 16 according to the present embodiment is an example of an image generation apparatus of the present disclosure, and an image generation method performed by the image processing apparatus 16 is an example of an image generation method of the present disclosure.

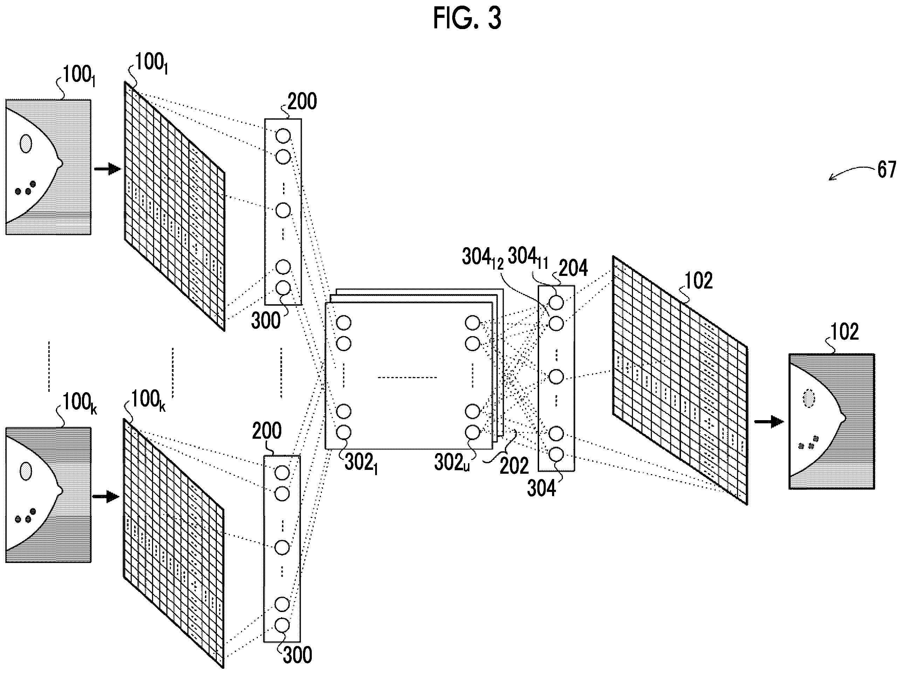

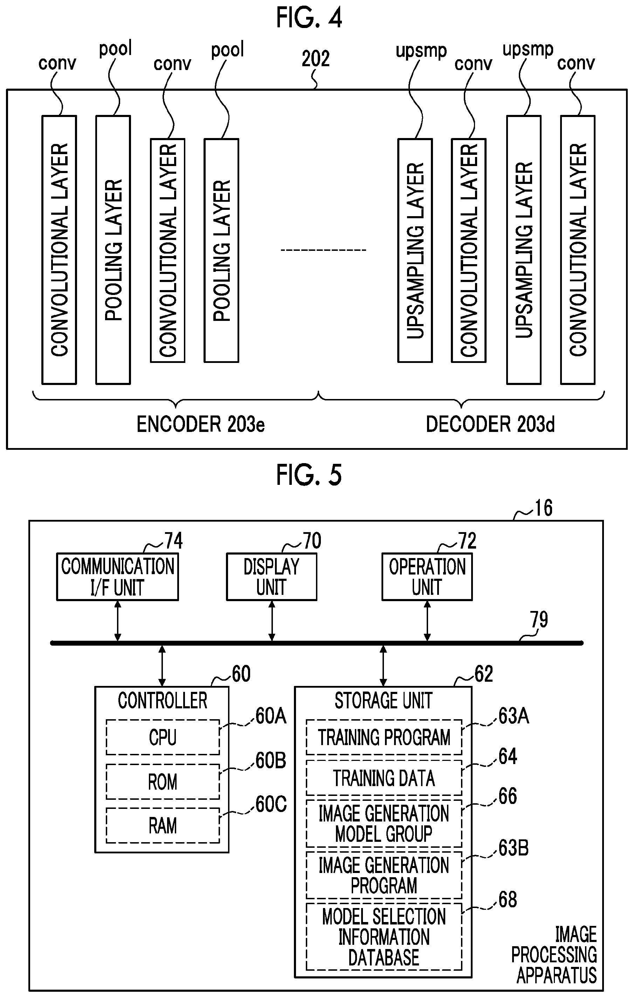

First, an example of an image generation model used for generating the pseudo two-dimensional image in the image processing apparatus 16 according to the present embodiment will be described. Fig. 3 shows an example of a plurality of image generation models (hereinafter, simply referred to as the "image generation model") 67 included in the image generation model group 66 (see also Fig. 5) according to the present embodiment. The image generation model 67 according to the present embodiment uses a convolutional neural network (CNN) that has been trained through machine learning by deep learning. The image generation model 67 receives, as input, a plurality of tomographic images 100 (in Fig. 3, k images: tomographic images 100₁ to 100ₖ) obtained from the series of the plurality of projection images, and outputs a pseudo two-dimensional image 102.

The image generation model 67 shown in Fig. 3 comprises an input layer 200, an intermediate layer 202, and an output layer 204 provided for each of the k tomographic images 100. It should be noted that, in the present embodiment, the input layer 200 provided for each tomographic image 100 has the same configuration.

The tomographic image 100 is input to the input layer 200. The input layer 200 includes a plurality of nodes 300, and each node 300 corresponds to each pixel of the tomographic image 100. The input layer 200 performs convolution processing in a case in which information for each pixel (each pixel) of the input tomographic image 100 is propagated to the intermediate layer 202. For example, in a case in which the size of the processing target image is 28 pixels × 28 pixels and the data is a grayscale, the size of the data propagated from the input layer 200 to the intermediate layer 202 is 28 × 28 × 1 = 784.

The present disclosure is not limited to the present embodiment, and a form may be adopted in which information in units of voxels cut out from the plurality of tomographic images 100 is input to the input layer 200.

As shown in Fig. 4, the intermediate layer 202 includes an encoder 203e and a decoder 203d. The encoder 203e includes a plurality of convolutional layers conv that perform the convolution processing and a plurality of pooling layers pool that perform pooling processing, in accordance with the number of layers of the encoder 203e (the number of layers shown in the encoder 203e in Fig. 4 is "2"). It should be noted that a plurality of nodes 302₁ shown in Fig. 3 correspond to a plurality of nodes included in the convolutional layer conv of the first layer included in the encoder 203e.

In the convolution processing, a three-dimensional convolution operation is performed on each pixel to output a pixel value Icp(x,y,z) corresponding to each pixel of interest Ip. In this way, three-dimensional output data Dc including a plurality of output data DIc having the pixel values Icp(x,y,z) arranged in a two-dimensional manner is output. One output is output to one filter F of 3 × 3 × 3 for the output data Dc. In a case in which a plurality of filters F having different types are used, the output data Dc is output for each filter F. The filter F means a neuron (node) of the convolutional layer, and the number of features that can be extracted from one input data D in the convolutional layer is the number of filters F because the extractable feature is determined for each filter F.

In addition, in the pooling layer pool, the pooling processing of reducing an original image while leaving the features is performed. In other words, in the pooling layer pool, the pooling processing of selecting a local representative value, reducing the resolution of the input image, and reducing the image size is performed. For example, in a case in which the pooling processing of selecting the representative value from the block of pixels of 2 × 2 is performed by shifting the stride by "1", that is, one pixel, a reduced image that is reduced to half the size of the input image is output.

Meanwhile, the decoder 203d includes a plurality of convolutional layers conv that perform the convolution processing and a plurality of upsampling layers upsmp that perform upsampling processing in accordance with the number of layers of the decoder 203d (the number of layers shown in the decoder 203d in Fig. 4 is "2"). It should be noted that a plurality of nodes 302ᵤ shown in Fig. 3 correspond to a plurality of nodes included in the convolutional layer conv of the first layer included in the decoder 203d.

The convolutional layer conv included in the decoder 203d performs the same processing as the convolutional layer conv included in the encoder 203e. Meanwhile, in the upsampling layer upsmp, the output of the encoder 203e is used as input, and processing is performed such that the image size of the output becomes the same as the image size of the pseudo two-dimensional image 102.

Meanwhile, the output layer 204 is a fully connected layer to which all the nodes 302ᵤ included in the convolutional layer conv disposed at the end of the intermediate layer 202 are connected. The image size in the output layer 204 is the same as the size of the pseudo two-dimensional image 102 output from the image generation model 67, and a plurality of nodes 304 included in the output layer 204 correspond to the pixels of the pseudo two-dimensional image 102.

As described above, in a case in which the plurality of tomographic images 100 are input, the image generation model 67 according to the present embodiment outputs the pseudo two-dimensional image 102.

Fig. 5 is a block diagram showing an example of a configuration of the image processing apparatus 16 according to the present embodiment. As shown in Fig. 5, the image processing apparatus 16 according to the present embodiment comprises a controller 60 as a computer, a storage unit 62, a display unit 70, an operation unit 72, and a communication I/F unit 74. The controller 60, the storage unit 62, the display unit 70, the operation unit 72, and the communication I/F unit 74 are connected via a bus 79, such as a system bus or a control bus, such that various types of information can be transmitted and received.

The controller 60 controls the overall operation of the image processing apparatus 16. The controller 60 comprises a CPU 60A as a processor, a ROM 60B, and a RAM 60C. Various programs and the like used by the CPU 60A for the control are stored in advance in the ROM 60B. The RAM 60C temporarily stores various types of data.

The storage unit 62 is a non-volatile storage unit and is, for example, an HDD or an SSD. The storage unit 62 stores various types of information, such as a training program 63A, an image generation program 63B, training data 64 for training the image generation model 67, the image generation model group 66 including the plurality of image generation models 67, and a model selection information database 68 described in detail later.

The display unit 70 displays the radiation images or various types of information. The display unit 70 is not particularly limited, and various displays and the like may be used. In addition, the operation unit 72 is used by the doctor to input instructions for a diagnosis for a lesion of the breast using the radiation image, the user to input various types of information, or the like. The operation unit 72 is not particularly limited, and examples of the operation unit 72 include various switches, a touch panel, a touch pen, and a mouse. The display unit 70 and the operation unit 72 may be integrated into a touch panel display.

The communication I/F unit 74 performs communication of various types of information between the console 12 and the PACS 14 via the network 17 by wireless communication or wired communication.

Next, the functions of the image processing apparatus 16 according to the present embodiment will be described. There are a training phase in which each of the image generation models 67 of the image generation model group 66 is trained and an operation phase in which the pseudo two-dimensional image is generated from the plurality of tomographic images by selectively using the image generation models 67 of the image generation model group 66.

### Training Phase

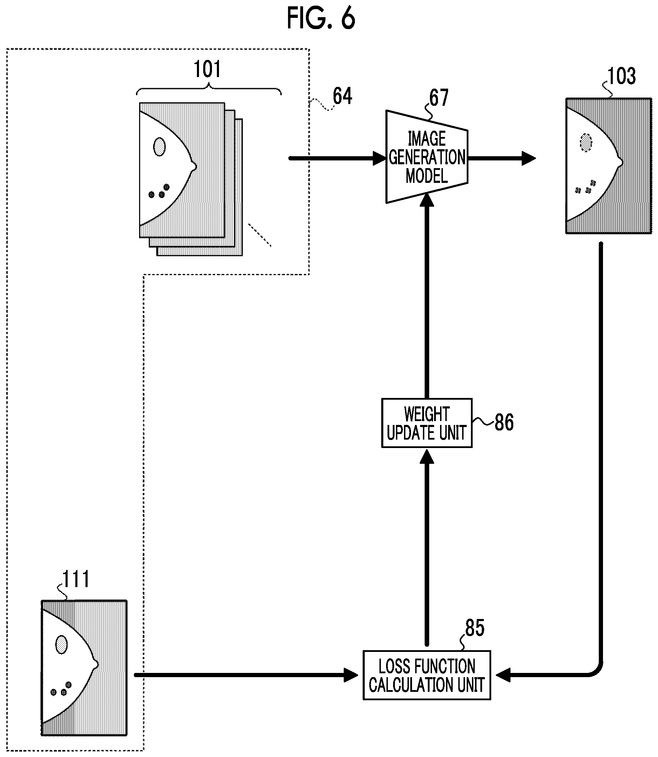

First, an example of a training phase of the image processing apparatus 16 according to the present embodiment will be described. Fig. 6 is a schematic diagram showing an outline of a flow of the training of each image generation model 67 of the image generation model group 66 in the image processing apparatus 16 according to the present embodiment.

As shown in Fig. 6, the training data 64 is composed of a set of a plurality of tomographic images 101 obtained from the series of the plurality of projection images obtained by the tomosynthesis imaging performed by the mammography apparatus 10 and a normal two-dimensional image 111 captured by the normal imaging by irradiating the breast U in a state of being compressed by the compression plate 30 during the tomosynthesis imaging with the radiation R. It should be noted that, in this case, the projection image and the normal two-dimensional image 111 may be captured in any order, that is, the projection image may be captured before or after the normal two-dimensional image 111. Hereinafter, a combination of the tomosynthesis imaging and the capturing of the normal two-dimensional image 111 in a state in which the same compression is performed by the compression plate 30 is referred to as "set imaging", and an image obtained by the set imaging is referred to as a "set of images".

In the training phase, the plurality of tomographic images 101 of the training data 64 are input to each image generation model 67 in the image generation model group 66. In response to this, the image generation model 67 outputs the pseudo two-dimensional image 103 as described above.

A loss function calculation unit 85 calculates a loss function, which is a function representing a degree of difference between the pseudo two-dimensional image 103 output from the image generation model 67 and the normal two-dimensional image 111. The closer the value of the loss function is to zero, the more similar the pseudo two-dimensional image 103 is to the normal two-dimensional image 111, and the more accurately the shape of the lesion is reproduced in the pseudo two-dimensional image 103.

A weight update unit 86 updates a weight of the network in the image generation model 67 in accordance with the loss function calculated by the loss function calculation unit 85. Specifically, a weight indicating the strength of the connection between the nodes of the previous and next layers, which are coefficients of each filter F in the image generation model 67, and a weight w of the difference in the connection between the nodes 304 of the output layer 204 in each tomographic image 101 to the nodes of the connection layer are changed by an error backpropagation method, a stochastic gradient descent method, or the like in accordance with the loss function calculated by the loss function calculation unit 85.

In the training phase, the series of processing of inputting the plurality of tomographic images 101 of the training data 64 to the image generation model 67, outputting the pseudo two-dimensional image 103 from the image generation model 67, calculating the loss function, and updating the weight is repeatedly performed to reduce the loss function.

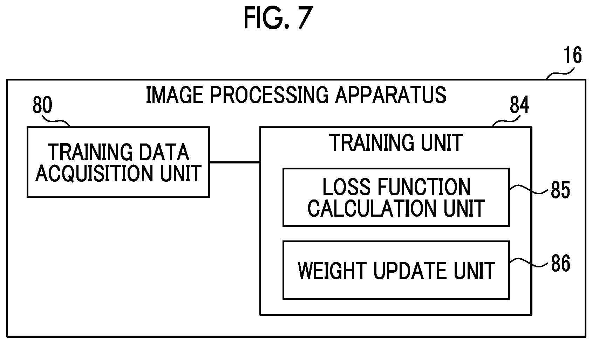

Fig. 7 is a functional block diagram showing an example of a configuration related to a function of training the image generation model 67 in the image processing apparatus 16 according to the present embodiment. As shown in Fig. 7, the image processing apparatus 16 comprises a training data acquisition unit 80 and a training unit 84. As an example, in the image processing apparatus 16 according to the present embodiment, the CPU 60A of the controller 60 executes the training program 63A stored in the storage unit 62, so that the CPU 60A functions as the training data acquisition unit 80 and the training unit 84.

The training data acquisition unit 80 has a function of acquiring the training data 64 from the storage unit 62. It should be noted that, in Fig. 6, a set of training data 64 is shown, but in practice, a sufficient amount of training data 64 for training the image generation model 67 is stored in the storage unit 62. The training data acquisition unit 80 outputs the acquired training data 64 to the training unit 84.

The training unit 84 includes the loss function calculation unit 85 and the weight update unit 86. The training unit 84 has a function of generating the image generation model 67 that receives a plurality of tomographic images 101 as input and outputs the pseudo two-dimensional image 103 by training a machine learning model through machine learning using the training data 64 as described above.

As described above, the loss function calculation unit 85 calculates the loss function representing the degree of difference between the pseudo two-dimensional image 103 output from the image generation model 67 and the normal two-dimensional image 111 of the training data 64.

As described above, the weight update unit 86 updates the weight of the network in the image generation model 67 in accordance with the loss function calculated by the loss function calculation unit 85.

The training unit 84 stores the generated image generation model 67 in the storage unit 62.

In the training phase according to the present embodiment, the plurality of image generation models 67 are trained by individually training the image generation model 67 for each predetermined category, and the plurality of image generation models 67 are stored in the storage unit 62 as the image generation model group 66.

Therefore, the training data 64 according to the present embodiment stores a set of the plurality of tomographic images 101 and the normal two-dimensional image 111 for each predetermined category (hereinafter, referred to as a "setting category"). Then, the training data acquisition unit 80 according to the present embodiment acquires the training data 64 from the storage unit 62 for each of the setting category, and the training unit 84 according to the present embodiment performs training of the image generation model 67 for each setting category, using the acquired training data 64. By the training for each setting category, the image generation model 67 for each setting category is generated.

In the present embodiment, a category related to the person under examination is applied as the setting category. It should be noted that, in the present embodiment, a height, a weight, a BMI, and an age of the person under examination are applied as the categories related to the person under examination, but the present disclosure is not limited thereto. For example, a form may be adopted in which a combination of a plurality of categories excluding any one of these categories or all the categories is applied as the setting category, and a form may be adopted in which a category related to the person under examination, which affects the pseudo two-dimensional image other than these categories, is applied as the setting category.

In addition, in a case in which the set of images, which is a combination of the plurality of tomographic images 101 and the normal two-dimensional image 111, is obtained by the set imaging in which the tomosynthesis imaging for obtaining the plurality of tomographic images 101 and the capturing of the normal two-dimensional image 111 corresponding to the tomographic image 101 are performed in the same compression state by the compression plate 30, the training unit 84 according to the present embodiment includes the set of images in the training data 64. As a result, in a case in which the set imaging is performed by the mammography apparatus 10, the set of images obtained by the set imaging can be used for the training of the image generation model 67.

Here, the training unit 84 according to the present embodiment accumulates the set of images by a predetermined amount and uses the set of images for the training of the image generation model 67, and sequentially updates the image generation model 67 using the set of images. As a result, it is possible to prevent excessive training from being performed by training the image generation model 67 each time the set of images is obtained.

In addition, in a case in which an image quality of at least one image in the combination of the plurality of tomographic images 101 and the normal two-dimensional image 111 is lower than a predetermined level, the training unit 84 according to the present embodiment prohibits the use of the images of the combination for the training. In the present embodiment, as a determination of whether or not the image quality of the image is lower than the predetermined level, both of a determination (hereinafter, referred to as a "first determination") of whether or not an amount of noise in the image is greater than a predetermined amount and a determination (hereinafter, referred to as a "second determination") of whether or not a body movement amount of the person under examination in the image is greater than a predetermined amount are applied.

It should be noted that, as a method of deriving the amount of noise of the image applied in the first determination, a known method in the related art disclosed in JP2016-190012A, JP2009-82498A, and the like can be applied. In addition, as a method of deriving the body movement amount of the person under examination in the image applied in the second determination, a known method in the related art disclosed in JP2022-125356A, JP2020-48991A, and the like can be applied. Therefore, further description of the method of deriving these physical quantities will be omitted.

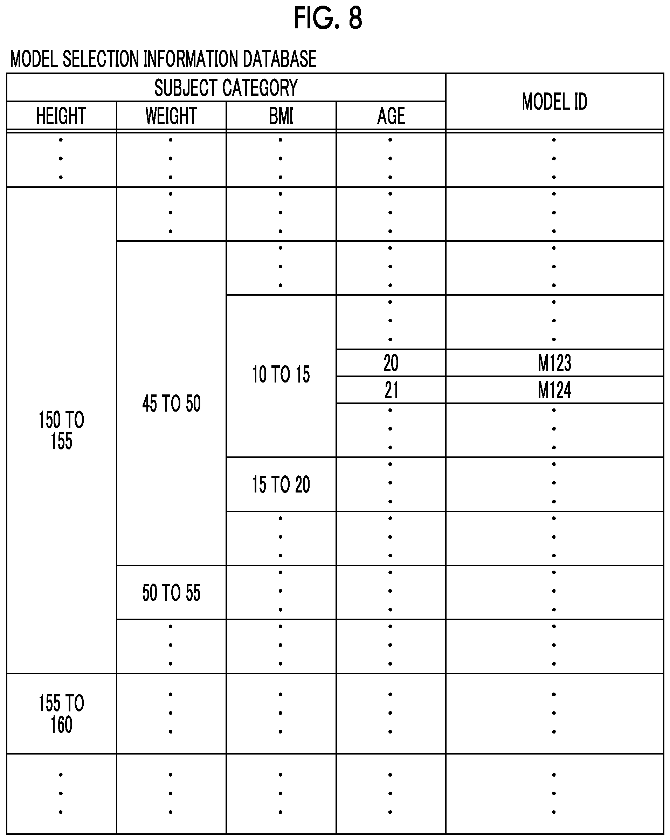

Next, the model selection information database 68 according to the present embodiment will be described with reference to Fig. 8. Fig. 8 is a schematic diagram showing an example of a configuration of the model selection information database 68 according to the present embodiment.

The model selection information database 68 according to the present embodiment is a database in which information for selectively applying the image generation model 67 generated for each setting category described above is registered. As shown in Fig. 8 as an example, in the model selection information database 68 according to the present embodiment, pieces of information on the person-under-examination category and the model identification (ID) are stored in association with each other.

The setting category is information indicating a difference in the above-described setting category, and is information including the height, the weight, the BMI, and the age of the person under examination as described above. Further, the model ID is information given in advance for each image generation model 67 in order to individually identify the plurality of image generation models 67 included in the image generation model group 66.

As described above, in the present embodiment, the model ID is applied as an identifier of the image generation model 67 included in the image generation model group 66. Therefore, the corresponding model ID is stored in association with each image generation model 67 included in the image generation model group 66.

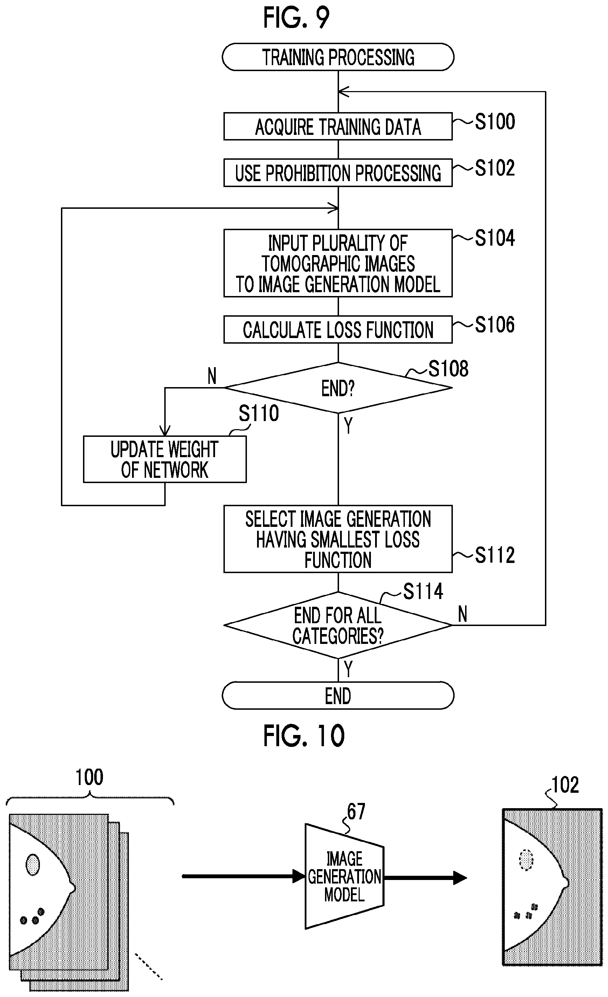

Next, an operation of the image processing apparatus 16 according to the present embodiment in the training phase will be described with reference to Fig. 9. The CPU 60A executes the training program 63A stored in the storage unit 62, to execute training processing shown in Fig. 9. It should be noted that, in order to avoid confusion, here, a case in which the set of images that is not used for the training for each setting category is accumulated in an amount equal to or greater than the predetermined amount at the start of execution of the present training processing will be described.

In step S100 of Fig. 9, as described above, the training data acquisition unit 80 acquires the training data 64 from the storage unit 62. In this case, the training data acquisition unit 80 acquires the training data 64 corresponding to any one category (hereinafter, referred to as an "applied category") in the setting category by reading out the training data 64 from the storage unit 62.

In next step S102, as described above, the training unit 84 performs the first determination and the second determination and determines whether or not the image having the image quality lower than the predetermined level is present among the plurality of tomographic images 101 and the normal two-dimensional image 111 in the training data 64 acquired in step S100. In a case in which the image having the image quality lower than a predetermined level is present, the training unit 84 executes use prohibition processing of deleting the combination of the plurality of tomographic images 101 and the normal two-dimensional image 111, including the image, to prohibit the use of the images of the combination for the training.

In next step S104, as described above, the training unit 84 inputs the plurality of tomographic images 101 included in the training data 64, on which the use prohibition processing of step S102 has been performed, to the image generation model 67 (hereinafter, referred to as an "applied model") corresponding to the applied category.

In next step S106, as described above, the loss function calculation unit 85 acquires the pseudo two-dimensional image 103 output from the applied model, and calculates the loss function representing the degree of difference between the pseudo two-dimensional image 103 output from the applied model and the corresponding normal two-dimensional image 111.

In next step S108, the training unit 84 determines whether or not to complete the training. In the present embodiment, the applied model having the smallest output of the loss function is finally adopted among the models that have been trained a predetermined number of times. Therefore, the training unit 84 determines whether or not the training is performed a predetermined number of times. Specifically, the training unit 84 determines whether or not the processing of steps S104 and S106 is performed a predetermined number of times. In a case in which the training is not performed a predetermined number of times, in other words, in a case in which the number of times the training is performed is less than the predetermined number of times, the training is not yet completed, so that a negative determination is made in the determination of step S108, and the processing proceeds to step S110.

In step S110, as described above, the weight update unit 86 updates the weight of the network in the applied model in accordance with the loss function calculated in step S106.

In a case in which the weight of the network of the applied model is updated by the processing of step S110, the processing returns to step S104, and the processing of steps S104 to S108 is repeated to perform the training again. As a result, a plurality of applied models corresponding to the number of times of training are obtained.

On the other hand, in a case in which the number of times of training is equal to or greater than the predetermined number of times, the training is completed, and a positive determination is made in the determination of step S108, and the processing proceeds to step S112.

In step S112, the training unit 84 selects, as the applied model finally obtained by the training, the applied model having the smallest loss function calculated in step S106 from among the plurality of applied models in accordance with the number of times of training.

In step S114, the training unit 84 determines whether or not the above-described processing is completed for all the categories in the setting category, returns to step S100 in a case in which a negative determination result is made, and completes the present training processing in a case in which a positive determination result is made. It should be noted that, in a case in which the processing of steps S100 to S114 is repeatedly executed, the training unit 84 sets a category that has not been set as the applied category in the setting category as the applied category.

By the above-described training processing, the training of the image generation model 67 corresponding to all the applied categories is performed.

It should be noted that the present training processing is not limited to the present training processing, and for example, a form may be adopted in which a threshold value is determined depending on whether or not the pseudo two-dimensional image 103 output from the image generation model 67 is sufficiently close to the normal two-dimensional image 111, and the training is completed in a case in which the output of the loss function is equal to or less than the threshold value, and the model in this case is set as the applied model obtained by the training.

In recent years, the number of facilities that perform only the tomosynthesis imaging or facilities that perform the tomosynthesis imaging and the mammography imaging separately has increased. In these facilities, in many cases, only an imaging apparatus that performs only the tomosynthesis imaging or only an imaging apparatus that performs the tomosynthesis imaging and the mammography imaging separately is disposed.

Therefore, although not shown, in the radiographic imaging system 1 according to the present embodiment, the imaging apparatuses are connected to the network 17, and the image generation model group 66 obtained by the training processing is transmitted to the imaging apparatus. In these imaging apparatuses, the image generation model 67 included in the image generation model group 66 is used for generating the pseudo two-dimensional image.

### Operation Phase

Next, an operation phase in which the pseudo two-dimensional image is generated using the image generation model 67 of the image generation model group 66 that has been trained as described above will be described.

Fig. 10 is a schematic diagram showing an outline of a flow of generating the pseudo two-dimensional image 102 using the image generation model 67 in the image processing apparatus 16 according to the present embodiment. As shown in Fig. 10, the image processing apparatus 16 generates the pseudo two-dimensional image 102 by inputting the plurality of tomographic images 100 to the image generation model 67 and causing the image generation model 67 to output the pseudo two-dimensional image 102. It should be noted that, in a case of operating the image generation model 67, the plurality of tomographic images 100 may be input in units of voxels, patches of the pseudo two-dimensional images 102 may be created, and the patches may be connected to generate the pseudo two-dimensional image 102.

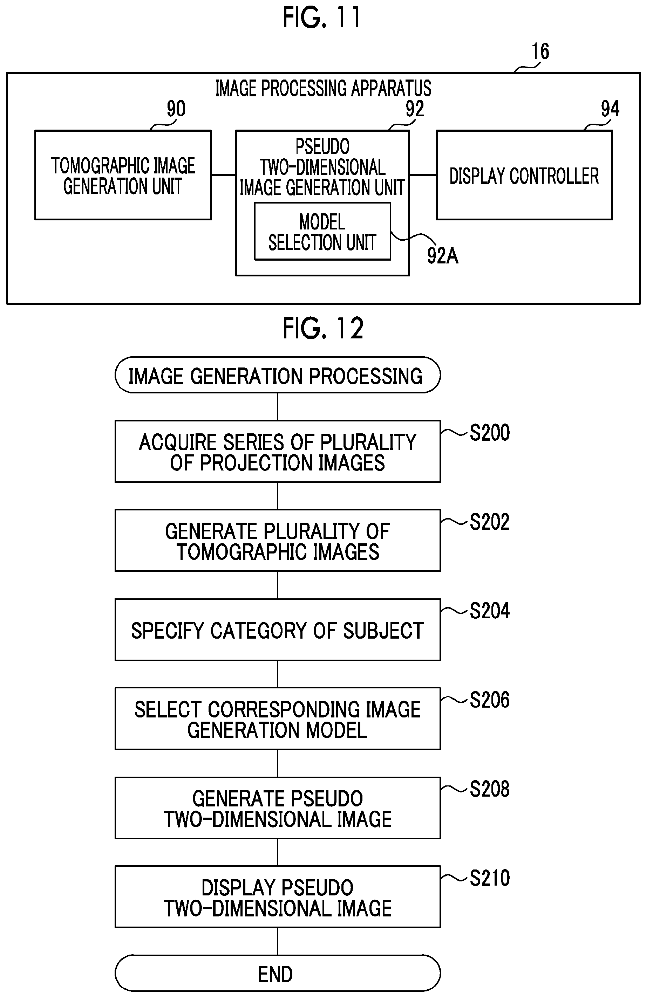

Fig. 11 is a functional block diagram showing an example of a configuration related to a function of generating the pseudo two-dimensional image 102 in the image processing apparatus 16. As shown in Fig. 11, the image processing apparatus 16 comprises a tomographic image generation unit 90, a pseudo two-dimensional image generation unit 92, and a display controller 94. As an example, in the image processing apparatus 16 according to the present embodiment, the CPU 60A of the controller 60 executes the image generation program 63B stored in the storage unit 62, so that the CPU 60A functions as the tomographic image generation unit 90, the pseudo two-dimensional image generation unit 92, and the display controller 94.

The tomographic image generation unit 90 has a function of generating the plurality of tomographic images from the series of the plurality of projection images. The tomographic image generation unit 90 acquires a desired series of the plurality of projection images from the console 12 of the mammography apparatus 10 or the PACS 14 based on an instruction to execute the generation of the pseudo two-dimensional image. Then, the tomographic image generation unit 90 generates the plurality of tomographic images 100 having different heights from the imaging surface 24A from the acquired series of the plurality of projection images. It should be noted that a method of generating, via the tomographic image generation unit 90, the plurality of tomographic images 100 is not particularly limited, and for example, the tomographic image generation unit 90 can generate the plurality of tomographic images 100 by reconstructing the series of the plurality of projection images by a back projection method such as a filtered back projection (FBP) method or a successive approximation reconstruction method. The tomographic image generation unit 90 outputs the plurality of generated tomographic images 100 to the pseudo two-dimensional image generation unit 92.

As shown in Fig. 10, the pseudo two-dimensional image generation unit 92 has a function of generating the pseudo two-dimensional image 102 using the image generation model 67. The pseudo two-dimensional image generation unit 92 inputs the plurality of tomographic images 100 to the image generation model 67. As a result, as described above, the pseudo two-dimensional image 102 is output from the image generation model 67. The pseudo two-dimensional image generation unit 92 acquires the pseudo two-dimensional image 102 output from the image generation model 67, and outputs the pseudo two-dimensional image 102 to the display controller 94.

Here, the pseudo two-dimensional image generation unit 92 according to the present embodiment comprises a model selection unit 92A. The model selection unit 92A selects the image generation model 67 corresponding to the category of the person under examination in the setting category from the image generation model group 66. Then, the pseudo two-dimensional image generation unit 92 generates the pseudo two-dimensional image 102 using the image generation model 67 selected by the model selection unit 92A.

The display controller 94 has a function of performing control of displaying the pseudo two-dimensional image 102 generated by the pseudo two-dimensional image generation unit 92 on the display unit 70.

Next, an operation of the generation of the pseudo two-dimensional image 102 in the image processing apparatus 16 according to the present embodiment will be described with reference to Fig. 12. The CPU 60A executes the image generation program 63B stored in the storage unit 62, to execute the image generation processing shown in Fig. 12.

In step S200 of Fig. 12, as described above, the tomographic image generation unit 90 acquires the series of the plurality of projection images from the console 12 or the PACS 14 of the mammography apparatus 10.

In next step S202, as described above, the tomographic image generation unit 90 generates the plurality of tomographic images 100 from the series of the plurality of projection images acquired in step S200.

In next step S204, the model selection unit 92A specifies the corresponding category of the person under examination. In step S206, the model selection unit 92A selectively acquires the model ID corresponding to the specified category from the model selection information database 68, to select the image generation model 67 corresponding to the specified category from the image generation model group 66. In the radiographic imaging system 1 according to the present embodiment, information indicating the category of the person under examination as a subject is registered in a header region of the tomographic image 100, and the category of the person under examination is specified by referring to the information, but the present disclosure is not limited to this form. For example, a form may be adopted in which the category of the person under examination is specified through a private tag of an image defined by digital imaging and communications in medicine (DICOM), which is a common standard for medical images. In addition, a form may be adopted in which the category of the person under examination is specified by acquiring the information indicating the category of the person under examination from a patient information management system (not shown) connected to the network 17.

In next step S208, as described above, the pseudo two-dimensional image generation unit 92 generates the pseudo two-dimensional image 102 using the selected image generation model 67. Specifically, the pseudo two-dimensional image 102, which is output from the image generation model 67, is acquired by inputting the plurality of tomographic images 100 generated in step S202 to the selected image generation model 67.

In next step S210, the display controller 94 performs control of displaying the pseudo two-dimensional image 102 obtained in step S208 on the display unit 70. It should be noted that a display form in which the pseudo two-dimensional image 102 is displayed on the display unit 70 is not particularly limited. For example, only the pseudo two-dimensional image 102 may be displayed on the display unit 70, or the plurality of tomographic images 100 and the pseudo two-dimensional image 102 may be displayed on the display unit 70. In a case in which the processing of step S210 is completed, the image generation processing shown in Fig. 12 is completed.

As described above, according to the present embodiment, as the image generation model, the model is applied, which has been trained in advance using the plurality of combinations of the plurality of tomographic images and the normal two-dimensional image captured by irradiating, with the radiation, the breast in a state of being compressed by the compression member during the tomosynthesis imaging for obtaining the plurality of tomographic images. Therefore, it is possible to improve the generation accuracy of the pseudo two-dimensional image as compared with a case of using an image generation model that has been trained using a combination of a plurality of tomographic images and a normal two-dimensional image captured separately from the tomosynthesis imaging for obtaining the plurality of tomographic images.

In addition, according to the present embodiment, the plurality of combinations of the plurality of tomographic images and the normal two-dimensional image are acquired for each predetermined category, and the image generation model is individually trained for each category, so that the plurality of image generation models are trained. Therefore, by using the plurality of image generation models in a differentiated manner for each category, it is possible to generate the pseudo two-dimensional image with higher accuracy than in a case in which the plurality of image generation models are not used in a differentiated manner.

In addition, according to the present embodiment, the category related to the person under examination is applied as the predetermined category. Therefore, it is possible to accurately generate the pseudo two-dimensional image corresponding to each category of the person under examination.

In particular, according to the present embodiment, as the category related to the person under examination, the category including at least one category of the height, the weight, the BMI, or the age of the person under examination is applied. Since the physique of the person under examination indicated by these categories affects the composition of the breast, it is possible to accurately generate the pseudo two-dimensional image corresponding to the category indicating the physique of the person under examination to which the pseudo two-dimensional image is applied.

In addition, according to the present embodiment, in a case in which the image quality of at least one image of the plurality of combinations of the plurality of tomographic images and the normal two-dimensional image is lower than a predetermined level, the use of the images of the combination for the training is prohibited. Therefore, it is possible to improve the generation accuracy of the pseudo two-dimensional image as compared with a case in which the prohibition is not performed.

In addition, according to the present embodiment, in a case in which the set imaging in which the tomosynthesis imaging and the capturing of the normal two-dimensional image are performed in the same compression state by the compression member is performed and the set of images which is a combination of the plurality of tomographic images and the normal two-dimensional image is obtained, the set of images is used as the training data of the image generation model. Accordingly, the training data of the image generation model can be obtained each time the set imaging is performed.

In particular, according to the present embodiment, the set of images is accumulated by a predetermined amount and used for training the image generation model, and the image generation model is sequentially updated using the set of images. Therefore, it is possible to prevent excessive training from being performed by training the image generation model each time the set of images is obtained.

In addition, according to the present embodiment, the plurality of tomographic images obtained from the series of the plurality of projection images obtained by the tomosynthesis imaging in a state in which the breast is compressed by the compression member are acquired as the images for generation of the pseudo two-dimensional image. Then, according to the present embodiment, the acquired image for generation is input to the image generation model to generate the pseudo two-dimensional image corresponding to the image for generation. Therefore, it is possible to improve the generation accuracy of the pseudo two-dimensional image as compared with a case of using, for the generation, the image generation model that has been trained using the combination of the plurality of tomographic images and the normal two-dimensional image captured separately from the tomosynthesis imaging for obtaining the plurality of tomographic images.

In particular, according to the present embodiment, the pseudo two-dimensional image is generated by selectively using the image generation model of the category corresponding to the pseudo two-dimensional image to be generated among the plurality of image generation models that have been individually trained for each predetermined category. Therefore, it is possible to generate the pseudo two-dimensional image with higher accuracy than in a case in which the plurality of image generation models are not used in a differentiated manner for each category.

Further, according to the present embodiment, the above-described image generation model is provided in the imaging apparatus that performs only the tomosynthesis imaging and the imaging apparatus that separately performs the tomosynthesis imaging and the mammography imaging. Therefore, even in these imaging apparatuses, it is possible to improve the generation accuracy of the pseudo two-dimensional image as compared with a case of using, for the generation, the image generation model that has been trained using the combination of the plurality of tomographic images and the normal two-dimensional image captured separately from the tomosynthesis imaging for obtaining the plurality of tomographic images.

It should be noted that, in the present embodiment, a case has been described in which at least one of the height, the weight, the BMI, or the age indicating the physique of the person under examination is applied as a predetermined category of the present disclosure, but the present disclosure is not limited thereto. For example, a form may be adopted in which at least one of mammary gland information indicating a type of a mammary gland of the breast of the person under examination or a thickness of the breast of the person under examination is applied as a predetermined category of the present disclosure.

That is, as disclosed in JP2019-58606A, the type of the mammary gland of the breast is classified into four types, that is, a high-density type, a fatty type, a scattered mammary gland type, and a heterogeneous high-density type. Therefore, by generating the image generation model separately for each of the four types of breast and applying the image generation model to the generation of the pseudo two-dimensional image, it is possible to accurately generate the pseudo two-dimensional image specialized for each type of breast. It should be noted that the type of the breast of the person under examination in this form can be specified by applying a known method in the related art, such as the method disclosed in JP2019-58606A, from a series of a plurality of corresponding projection images or the plurality of tomographic images.

In addition, in a case in which the thickness of the breast is different, a tube voltage of the tube in the radiation source 29 during the imaging is different, or the amount of scattered rays generated by the radiation is different. Therefore, by generating the image generation model for each thickness of the breast and applying the image generation model to the generation of the pseudo two-dimensional image, it is possible to accurately generate the pseudo two-dimensional image in accordance with the thickness of the breast.

### Second Embodiment

In the first embodiment, the category related to the person under examination is applied as the predetermined category of the present disclosure. On the other hand, in the present embodiment, a category related to a setting content during the imaging is applied as a predetermined category of the present disclosure. In particular, in the present embodiment, the irradiation angle of the radiation and the resolution of the captured image during the tomosynthesis imaging are applied as the categories related to the setting content during the imaging.

That is, as disclosed in JP2014-166357A as an example, the irradiation angle of the radiation and the resolution of the captured image during the tomosynthesis imaging may be different for each of a plurality of types of imaging modes determined in advance.

For example, in the example disclosed in JP2014-166357A, a diagnosis mode and an imaging mode are provided as the imaging modes, and the user such as the doctor can select the imaging mode. The diagnosis mode is a mode in which the subject is roughly imaged in order for the user to perform a medical examination, a diagnosis, or the like, and the imaging is performed for each degree in a range of +10 degrees to -10 degrees as an example. In addition, the imaging mode is a mode for performing the imaging with higher definition than imaging in the diagnosis mode, and the imaging is performed for each degree in a range of +20 degrees to -20 degrees as an example. As described above, in this example, in a case of performing imaging for obtaining a high-definition image, the imaging is performed by setting the angle to be greater in order to increase an amount of information (image information amount).

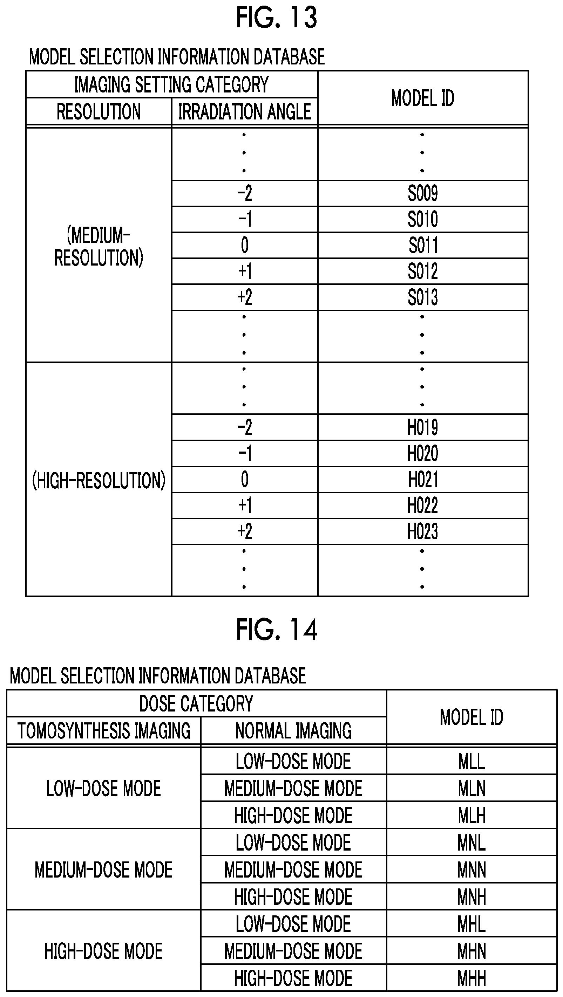

Therefore, in the image processing apparatus 16 according to the present embodiment, categories of the irradiation angle of the radiation and the resolution of the captured image during the tomosynthesis imaging are applied as the setting categories, which are the categories of the image generation model 67. It should be noted that the configuration of the radiographic imaging system 1 according to the present embodiment is substantially the same as that of the first embodiment except for the configuration of the model selection information database 68, and thus the model selection information database 68 according to the present embodiment will be described below with reference to Fig. 13. Fig. 13 is a schematic diagram showing an example of a configuration of the model selection information database 68 according to the present embodiment.

The model selection information database 68 according to the present embodiment is a database in which information for selectively applying the image generation model 67 generated for each setting category is registered, and as shown in Fig. 13 as an example, pieces of information on an imaging setting category and the model ID are stored in association with each other.

The imaging setting category is information indicating a difference in the setting category, and includes pieces of information on the resolution of the captured image and the irradiation angle of the radiation as described above. It should be noted that the model ID is the same information as the model ID of the model selection information database 68 according to the first embodiment. In the example shown in Fig. 13, a case has been described in which a two-stage resolution, that is, a medium-resolution and a high-resolution is applied as the resolution and an angle in increments of 1 degree is applied as the irradiation angle, but the present disclosure is not limited thereto.

Therefore, in the training phase in the image processing apparatus 16 according to the present embodiment, a plurality of image generation models 67 that have been trained for each combination of the resolution and the irradiation angle indicated by the imaging setting category are generated. Further, in the operation phase of the image processing apparatus 16 according to the present embodiment, the image generation model 67 is used to generate the pseudo two-dimensional image 102 for each combination of the imaging setting categories.

It should be noted that, in the radiographic imaging system 1 according to the present embodiment, the information indicating the resolution and the irradiation angle is registered in the header region of the tomographic image 100, and the setting category of the tomographic image 100 is specified by referring to the information, but the present disclosure is not limited to this form. For example, a form may be adopted in which the setting category is specified through the private tag of the image defined in the DICOM. In addition, a form may be adopted in which the setting category is specified by acquiring the information indicating the resolution and the irradiation angle from the patient information management system (not shown) connected to the network 17.

As described above, according to the present embodiment, the category related to the setting content during the imaging is applied as the predetermined category. Therefore, it is possible to accurately generate the pseudo two-dimensional image corresponding to the category of the setting content during the imaging.

In particular, according to the present embodiment, the irradiation angle of the radiation and the resolution of the captured image during the tomosynthesis imaging are applied as the categories related to the setting content during the imaging. Therefore, it is possible to accurately generate the pseudo two-dimensional image corresponding to the irradiation angle and the resolution of the radiation.

It should be noted that, in the present embodiment, a case has been described in which both the irradiation angle of the radiation and the resolution of the captured image during the tomosynthesis imaging are combined and applied as the category related to the setting content during the imaging, but the present disclosure is not limited thereto. For example, a form may be adopted in which only one of the irradiation angle of the radiation or the resolution of the captured image during the tomosynthesis imaging is applied as the category related to the setting content during the imaging.

### Third Embodiment

In the second embodiment, the category related to the setting content during the imaging is applied as a predetermined category of the present disclosure, and at least one of the irradiation angle of the radiation or the resolution of the captured image during the tomosynthesis imaging is applied as the category related to the setting content during the imaging. On the other hand, in the present embodiment, a dose mode of the radiation is applied as the category related to the setting content during the imaging.

That is, during the tomosynthesis imaging and the capturing of the normal two-dimensional image, the dose mode in which the dose of the radiation emitted during the imaging is classified into a plurality of stages is often applicable.

Therefore, in the image processing apparatus 16 according to the present embodiment, categories of the dose modes during the tomosynthesis imaging and the capturing of the normal two-dimensional image are applied as the setting categories, which are the categories of the image generation model 67. It should be noted that the configuration of the radiographic imaging system 1 according to the present embodiment is substantially the same as that of the first embodiment except for the configuration of the model selection information database 68, and thus the model selection information database 68 according to the present embodiment will be described below with reference to Fig. 14. Fig. 14 is a schematic diagram showing an example of a configuration of the model selection information database 68 according to the present embodiment.

The model selection information database 68 according to the present embodiment is a database in which information for selectively applying the image generation model 67 generated for each setting category is registered, and as shown in Fig. 14 as an example, pieces of information on a dose category and the model ID are stored in association with each other.

The above-described dose category is information indicating the difference in the setting category, and as described above, includes information indicating a difference in the dose mode of the tomosynthesis imaging and information indicating a difference in the dose mode in the capturing of the normal two-dimensional image. It should be noted that the model ID is the same information as the model ID of the model selection information database 68 according to the first embodiment. In the example shown in Fig. 14, a case is shown in which three stages of modes, that is, a low-dose mode, a medium-dose mode, and a high-dose mode are applied as the dose mode, but the present disclosure is not limited thereto.

Therefore, in the training phase in the image processing apparatus 16 according to the present embodiment, the plurality of image generation models 67 that have been trained for each combination of the dose mode during the tomosynthesis imaging indicated by the dose category and the dose mode during the capturing of the normal two-dimensional image are generated. Further, in the operation phase of the image processing apparatus 16 according to the present embodiment, the image generation model 67 is used to generate the pseudo two-dimensional image 102 for each combination of the dose categories.

It should be noted that, in the radiographic imaging system 1 according to the present embodiment, information indicating the dose mode applied during the imaging is registered in the header regions of the tomographic image 100 and the normal two-dimensional image 111, and the dose modes of the tomographic image 100 and the normal two-dimensional image 111 are specified by referring to the information, but the present disclosure is not limited to this form. For example, a form may be adopted in which the dose mode is specified through the private tag of the image defined in the DICOM. In addition, a form may be adopted in which the dose mode is specified by acquiring the information indicating the dose mode from the patient information management system (not shown) connected to the network 17.

As described above, according to the present embodiment, the dose mode of the radiation is applied as the category related to the setting content during the imaging. Therefore, it is possible to accurately generate the pseudo two-dimensional image corresponding to the dose mode of the radiation during the imaging.

It should be noted that, in the present embodiment, as the category related to the setting content during the imaging, a case has been described in which all combinations of the dose mode during the tomosynthesis imaging and the dose mode during the capturing of the normal two-dimensional image are applied, but the present disclosure is not limited thereto.

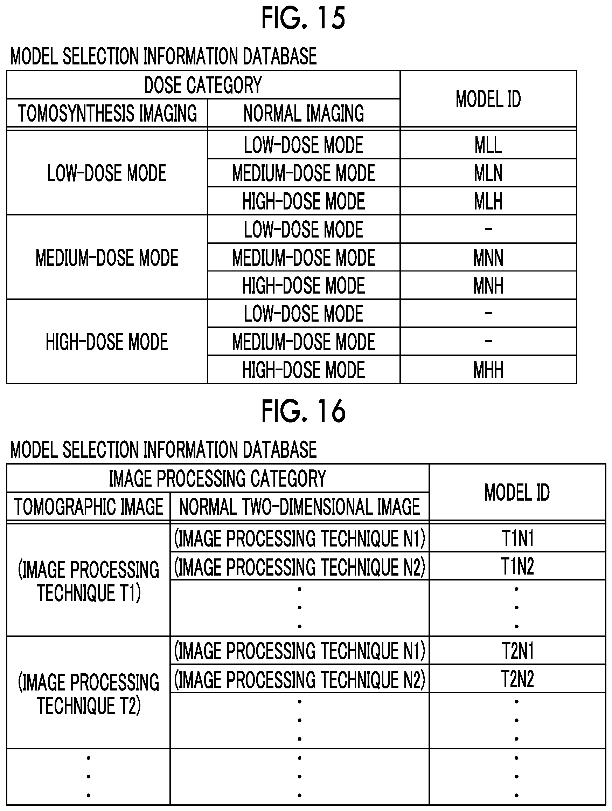

For example, in a case in which the pseudo two-dimensional image is generated from the series of the plurality of tomographic images, it may be desired to set the image quality of the pseudo two-dimensional image to be equal to or higher than the image quality of the tomographic image. In this case, as shown in Fig. 15, for the plurality of stages of the dose modes during the tomosynthesis imaging, the image generation model 67 corresponding only to a dose mode having a dose equal to or greater than the dose of the corresponding tomosynthesis imaging as the dose mode during the capturing of the normal two-dimensional image may be prepared. In this form, the dose mode during the tomosynthesis imaging shown in Fig. 15 corresponds to a first dose mode of the present disclosure, and the dose mode during the capturing of the normal two-dimensional image shown in Fig. 15 corresponds to a second dose mode having a dose equal to or greater than the first dose mode of the present disclosure. Fig. 15 is a schematic diagram showing an example of another configuration of the model selection information database 68 according to the present embodiment.

According to this form, it is possible to reduce the number of image generation models as compared with a case of generating an image generation model corresponding to all combinations of the dose mode during the tomosynthesis imaging and the dose mode during the capturing of the normal two-dimensional image.

### Fourth Embodiment

In each of the above-described embodiments, the category related to the person under examination or the category related to the setting content during the imaging is applied as a predetermined category of the present disclosure. On the other hand, in the present embodiment, the category related to the type of the image processing technique for at least one of the plurality of tomographic images or the normal two-dimensional image is applied as a predetermined category of the present disclosure.

That is, the above-described series of the plurality of projection images, the plurality of tomographic images obtained from the projection images, and the normal two-dimensional image are generally subjected to unique image processing for each manufacturer of the mammography apparatus 10. It should be noted that examples of the image processing referred to here include image processing of reducing noise in the image and image processing of sharpening the image.

Therefore, in the image processing apparatus 16 according to the present embodiment, category related to the types of the image processing techniques for the tomographic image 100 and the normal two-dimensional image 111 is applied as the setting category, which are the categories of the image generation model 67. It should be noted that the configuration of the radiographic imaging system 1 according to the present embodiment is substantially the same as that of the first embodiment except for the configuration of the model selection information database 68, and thus the model selection information database 68 according to the present embodiment will be described below with reference to Fig. 16. Fig. 16 is a schematic diagram showing an example of a configuration of the model selection information database 68 according to the present embodiment.

The model selection information database 68 according to the present embodiment is a database in which information for selectively applying the image generation model 67 generated for each setting category is registered, and as shown in Fig. 16 as an example, pieces of information on an image processing category and the model ID are stored in association with each other.

The image processing category is information indicating a difference in the setting category, and includes information indicating a difference in a type of the image processing technique applied to the tomographic image 100 and information indicating a difference in a type of the image processing technique applied to the normal two-dimensional image 111, as described above. It should be noted that the model ID is the same information as the model ID of the model selection information database 68 according to the first embodiment.

Therefore, in the training phase in the image processing apparatus 16 according to the present embodiment, the plurality of image generation models 67 that have been trained for each combination of the image processing technique for the tomographic image 100 and the image processing technique for the normal two-dimensional image 111, which are indicated by the image processing categories, are generated. Further, in the operation phase of the image processing apparatus 16 according to the present embodiment, the image generation model 67 is used to generate the pseudo two-dimensional image 102 for each combination of the image processing categories.

It should be noted that, in the radiographic imaging system 1 according to the present embodiment, information indicating the applied image processing technique is registered in the header regions of the tomographic image 100 and the normal two-dimensional image 111, and the image processing techniques of the tomographic image 100 and the normal two-dimensional image 111 are specified by referring to the information, but the present disclosure is not limited to this form. For example, a form may be adopted in which the image processing technique is specified through the private tag of the image defined by the DICOM. In addition, a form may be adopted in which the image processing technique is specified by acquiring the information indicating each image processing technique from the patient information management system (not shown) connected to the network 17.

As described above, according to the present embodiment, the category related to the types of the image processing techniques for the plurality of tomographic images and the normal two-dimensional image is applied as the predetermined category. Therefore, the pseudo two-dimensional image corresponding to the type of the applied image processing technique can be accurately generated.

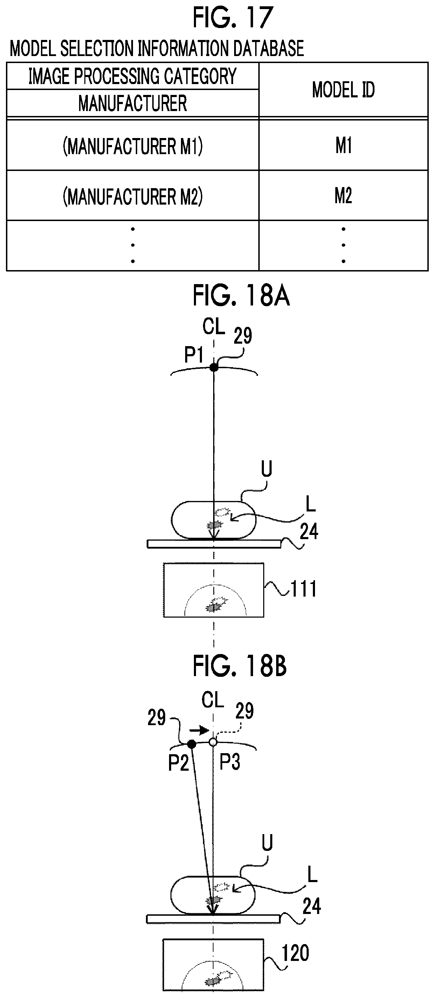

It should be noted that, in the present embodiment, a case has been described in which the category related to the types of the image processing techniques for the plurality of tomographic images and the normal two-dimensional image is applied as the predetermined category, but the present disclosure is not limited thereto. For example, as shown in Fig. 17 as an example, a form may be adopted in which a category for each manufacturer of the mammography apparatus 10 that performs the tomosynthesis imaging is applied as the category related to the type of the image processing technique. Fig. 17 is a schematic diagram showing an example of another configuration of the model selection information database 68 according to the present embodiment.

According to this form, the number of image generation models can be reduced as compared with a case in which the category related to the type of the image processing technique for the plurality of tomographic images and the normal two-dimensional image is applied as the predetermined category.

### Fifth Embodiment

During the tomosynthesis imaging and the capturing of the normal two-dimensional image, that is, the normal imaging, a position of the tube of the radiation source 29 (in the example shown in Fig. 2, the position on a normal line CL, hereinafter, referred to as a "reference position") during the imaging may be shifted. In this case, even in a case in which the breast of the same person under examination is imaged by the set imaging, the shifting occurs in the position of the same tissue, such as calcification or mammary gland, between the projection image or tomographic image obtained by the tomosynthesis imaging and the normal two-dimensional image.

Fig. 18A is a diagram showing an example of an irradiation position P1 of the radiation and the normal two-dimensional image 111 in a case in which the normal imaging is performed. Fig. 18B is a diagram showing an example of an irradiation position P2 of the radiation (that is, a position of the irradiation position 19₄ in Fig. 2) and a projection image 120 in a case of being shifted from the reference position during the normal imaging in a case in which the tomosynthesis imaging is performed.

As shown in Fig. 18B, it can be seen that a positional relationship of a lesion L is changed in the projection image 120 obtained by performing the tomosynthesis imaging on the breast U in a state in which the reference position is shifted from the irradiation position P1 to the irradiation position P2 during the normal imaging, as compared with the normal two-dimensional image 111 shown in Fig. 18A. Therefore, in a case in which the tomographic image 101 is generated using the series of the plurality of projection images obtained in this state, the positional relationship of the lesion L shown by the tomographic image 101 is also changed from that of the normal two-dimensional image 111.

Meanwhile, in a case in which the plurality of tomographic images 101 and the corresponding normal two-dimensional image 111 are used for training the image generation model 67, the higher the correlation, the higher the generation accuracy of the pseudo two-dimensional image.

Therefore, in the training unit 84 of the image processing apparatus 16 according to the present embodiment, the plurality of tomographic images 101 used for training the image generation model 67 are corrected to be tomographic images in a case in which the tube is virtually provided at the position of the tube that emits the radiation in a case of capturing the corresponding normal two-dimensional image 111.

As shown in Fig. 18B as an example, the training unit 84 according to the present embodiment derives a corresponding virtual projection position P3 from the irradiation position P1 during the normal imaging in order to match the positional relationship of the lesion L during the tomosynthesis imaging with the normal two-dimensional image 111. Specifically, for example, a difference between the irradiation position P1 and the irradiation position P2 may be obtained, and the irradiation position during the tomosynthesis imaging may be corrected in accordance with the obtained difference. The virtual projection position P3 is derived for each irradiation position during the tomosynthesis imaging based on the obtained difference. The virtual projection position P3 is a position at which the breast U is virtually projected during the tomosynthesis imaging.

The training unit 84 generates the tomographic image 101 in which the correction has been performed, based on the derived virtual projection position P3. In this case, the training unit 84 generates the plurality of tomographic images 101 of which the magnification ratios have been corrected using the virtual projection position P3 as a center.

In the radiographic imaging system 1 according to the present embodiment, information indicating the irradiation position applied during the imaging is registered in the header regions of the tomographic image 101 and the normal two-dimensional image 111, and the irradiation position is specified by referring to the information, but the present disclosure is not limited to this form. For example, a form may be adopted in which the irradiation position is specified through the private tag of the image defined by the DICOM. In addition, the irradiation position may be specified by acquiring the information indicating the irradiation position from the patient information management system (not shown) connected to the network 17.

It should be noted that the operations of the training phase and the operation phase of the image processing apparatus 16 according to the present embodiment are the same as those of the first embodiment except that the tomographic images 101 used for training the image generation model 67 are corrected as described above, and thus the description thereof will be omitted here.

As described above, according to the present embodiment, in a case in which the plurality of tomographic images are used for the training, the plurality of tomographic images are corrected to be tomographic images in a case in which the tube is virtually provided at the position of the tube that emits the radiation in a case of capturing the corresponding normal two-dimensional image. Therefore, it is possible to generate the pseudo two-dimensional image with higher accuracy than in a case in which the correction is not performed.

It should be noted that, in each of the above-described embodiments, a case has been described in which the CPU 60A provided in the image processing apparatus 16 is applied as a processor of the technology of the present disclosure, but the present disclosure is not limited thereto. For example, a CPU of a controller provided in the mammography apparatus 10 or a CPU of the controller 40 provided in the console 12 may be applied as a processor of the technology of the present disclosure.

In addition, in each of the above-described embodiments, a case has been described in which the plurality of tomographic images are applied as the information to be input to the image generation model 67 during the training phase and the operation phase, but the present disclosure is not limited thereto. For example, a form may be adopted in which the series of the plurality of projection images obtained by the tomosynthesis imaging is applied as the information to be input to the image generation model 67. In this case, the training of the image generation model 67 can be performed or the pseudo two-dimensional image can be generated without generating the tomographic image.

In addition, in each of the above-described embodiments, a case has been described in which the model based on the CNN is applied as the image generation model 67, but the present disclosure is not limited thereto. For example, as the image generation model 67, a model using a recurrent neural network (RNN) or a model using other generation artificial intelligence (AI) may be applied.

In addition, in each of the above-described embodiments, a case has been described in which the image generation model 67 is provided in the image processing apparatus 16, but the present disclosure is not limited thereto. For example, a form may be adopted in which the image generation model 67 is provided in the mammography apparatus 10.

In addition, in each of the above-described embodiments, for example, as a hardware structure of processing units that execute various types of processing, such as the training data acquisition unit 80, the training unit 84, the loss function calculation unit 85, the weight update unit 86, the tomographic image generation unit 90, the pseudo two-dimensional image generation unit 92, the model selection unit 92A, and the display controller 94, various processors shown below can be used. As described above, in addition to the CPU that is a general-purpose processor that executes software (program) to function as various processing units, the various processors include a programmable logic device (PLD) that is a processor whose circuit configuration can be changed after manufacture, such as a field programmable gate array (FPGA), and a dedicated electric circuit that is a processor having a circuit configuration that is designed for exclusive use in order to execute specific processing, such as an application specific integrated circuit (ASIC).

One processing unit may be configured by one of the various processors or may be configured by a combination of two or more processors of the same type or different types (for example, a combination of a plurality of FPGAs or a combination of a CPU and an FPGA). Moreover, a plurality of processing units may be configured by one processor.

A first example of the configuration in which the plurality of processing units are configured by one processor is a form in which one processor is configured by a combination of one or more CPUs and the software and this processor functions as the plurality of processing units, as represented by computers such as a client and a server. A second example is a form of using a processor that implements the function of the entire system including the plurality of processing units via one integrated circuit (IC) chip, as represented by a system on a chip (SoC) or the like. As described above, as the hardware structure, the various processing units are configured by one or more of the various processors described above.

Further, as the hardware structure of the various processors, more specifically, an electric circuit (circuitry) in which circuit elements such as semiconductor elements are combined can be used.

In addition, in each of the above-described embodiments, an aspect has been described in which each program of the training program 63A and the image generation program 63B is stored (installed) in advance in the storage unit 62 of the image processing apparatus 16, but the present disclosure is not limited to this. Each program described above may be provided in a form of being recorded on a recording medium, such as a compact disc read-only memory (CD-ROM), a digital versatile disc read-only memory (DVD-ROM), and a universal serial bus (USB) memory. In addition, each program described above may have a form being downloaded from the external device via the network.

Further, the present invention can also be applied to a program and a program product.

From the above description, the invention according to the following supplementary notes can be understood.

### Supplementary Note 1

An image generation apparatus that generates a pseudo two-dimensional image from a series of a plurality of projection images obtained by tomosynthesis imaging in a state in which a breast is compressed by a compression member or a plurality of tomographic images obtained from the series of the plurality of projection images, the image generation apparatus comprising: at least one image generation model, in which the image generation model has been trained in advance using a plurality of combinations of the plurality of projection images or the plurality of tomographic images and a normal two-dimensional image captured by irradiating, with radiation, the breast in a state of being compressed by the compression member during the tomosynthesis imaging for obtaining the plurality of projection images or the plurality of tomographic images.

### Supplementary Note 2

The image generation apparatus according to supplementary note 1, further comprising: a processor, in which the processor is configured to: acquire the plurality of combinations of the plurality of projection images or the plurality of tomographic images and the normal two-dimensional image, for each predetermined category; and train a plurality of the image generation models by individually training the image generation model for each category.

### Supplementary Note 3

The image generation apparatus according to supplementary note 2, in which the predetermined category is a category related to a person under examination.

### Supplementary Note 4

The image generation apparatus according to supplementary note 3, in which the category related to the person under examination includes at least one category of a height, a weight, a BMI, an age, mammary gland information, or a thickness of a breast of the person under examination.

### Supplementary Note 5

The image generation apparatus according to supplementary note 2, in which the predetermined category is a category related to a setting content during imaging.

### Supplementary Note 6

The image generation apparatus according to supplementary note 5, in which the category related to the setting content during the imaging includes at least one category of an irradiation angle of the radiation, a resolution of a captured image, or a dose mode of the radiation during the tomosynthesis imaging.

### Supplementary Note 7

The image generation apparatus according to supplementary note 6, in which the category of the dose mode of the radiation is a category for each combination of a first dose mode during the tomosynthesis imaging and a second dose mode that is a dose mode in a case of capturing the normal two-dimensional image and that has a dose equal to or greater than a dose in the first dose mode.

### Supplementary Note 8

The image generation apparatus according to supplementary note 2, in which the predetermined category is a category related to a type of an image processing technique for at least one of the plurality of projection images or the plurality of tomographic images or the normal two-dimensional image.

### Supplementary Note 9

The image generation apparatus according to supplementary note 8, in which the category related to the type of the image processing technique is a category for each manufacturer of an imaging apparatus that performs the tomosynthesis imaging.

### Supplementary Note 10

The image generation apparatus according to any one of supplementary notes 2 to 9, in which the processor is configured to: in a case in which an image quality of at least one image in the plurality of combinations of the plurality of projection images or the plurality of tomographic images and the normal two-dimensional image is lower than a predetermined level, prohibit use of the images of the combination for the training.

### Supplementary Note 11

The image generation apparatus according to any one of supplementary notes 2 to 10, in which the processor is configured to: in a case in which the plurality of tomographic images are used for the training, correct the plurality of tomographic images to tomographic images in a case of virtually providing a tube at a position of the tube that emits the radiation in a case of capturing a corresponding normal two-dimensional image.

### Supplementary Note 12

The image generation apparatus according to any one of supplementary notes 2 to 11, in which the processor is configured to: in a case in which a set of images, which is the combination of the plurality of projection images or the plurality of tomographic images and the normal two-dimensional image, is obtained by set imaging in which the tomosynthesis imaging and the capturing of the normal two-dimensional image are performed in the same compression state by the compression member, use the set of images as training data for the image generation model.

### Supplementary Note 13

The image generation apparatus according to supplementary note 12, in which the processor is configured to: accumulate the set of images and use the set of images for the training of the image generation model.

### Supplementary Note 14

The image generation apparatus according to supplementary note 12 or 13, in which the processor is configured to: sequentially update the image generation model using the set of images.

### Supplementary Note 15

The image generation apparatus according to any one of supplementary notes 2 to 14, in which the processor is configured to: acquire the series of the plurality of projection images obtained by the tomosynthesis imaging in a state in which the breast is compressed by the compression member or the plurality of tomographic images obtained from the series of the plurality of projection images, as images for generation of the pseudo two-dimensional image; and input the acquired images for generation to the image generation model to generate the pseudo two-dimensional image corresponding to the images for generation.

### Supplementary Note 16

The image generation apparatus according to supplementary note 15, in which the processor is configured to: in a case in which the plurality of image generation models that have been individually trained for each predetermined category are provided as the image generation model, generate the pseudo two-dimensional image by selectively using the image generation model of the category corresponding to the pseudo two-dimensional image to be generated among the plurality of image generation models.

### Supplementary Note 17

The image generation apparatus according to any one of supplementary notes 1 to 16, in which the image generation model is provided in an imaging apparatus that performs only the tomosynthesis imaging or an imaging apparatus that separately performs the tomosynthesis imaging and mammography imaging.

### Supplementary Note 18

An image generation method executed by a computer, the image generation method comprising: generating, via an image generation model, a pseudo two-dimensional image from a series of a plurality of projection images obtained by tomosynthesis imaging in a state in which a breast is compressed by a compression member or a plurality of tomographic images obtained from the series of the plurality of projection images, in which the image generation model has been trained in advance using a plurality of combinations of the plurality of projection images or the plurality of tomographic images and a normal two-dimensional image captured by irradiating, with radiation, the breast in a state of being compressed by the compression member during the tomosynthesis imaging for obtaining the plurality of projection images or the plurality of tomographic images.

### Supplementary Note 19

A program causing a computer to execute a process comprising: generating, via an image generation model, a pseudo two-dimensional image from a series of a plurality of projection images obtained by tomosynthesis imaging in a state in which a breast is compressed by a compression member or a plurality of tomographic images obtained from the series of the plurality of projection images, in which the image generation model has been trained in advance using a plurality of combinations of the plurality of projection images or the plurality of tomographic images and a normal two-dimensional image captured by irradiating, with radiation, the breast in a state of being compressed by the compression member during the tomosynthesis imaging for obtaining the plurality of projection images or the plurality of tomographic images.

## Claims

1. An image generation apparatus (16) that generates a pseudo two-dimensional image (102; 103) from a series of a plurality of projection images obtained by tomosynthesis imaging in a state in which a breast (U) is compressed by a compression member (30) or a plurality of tomographic images (100; 101) obtained from the series of the plurality of projection images, the image generation apparatus comprising:
at least one image generation model (67),
wherein the image generation model has been trained in advance using a plurality of combinations of the plurality of projection images or the plurality of tomographic images and a normal two-dimensional image (111) captured by irradiating, with radiation (R), the breast in a state of being compressed by the compression member during the tomosynthesis imaging for obtaining the plurality of projection images or the plurality of tomographic images.

2. The image generation apparatus according to claim 1, further comprising:
a processor (60A),
wherein the processor is configured to:
acquire the plurality of combinations of the plurality of projection images or the plurality of tomographic images and the normal two-dimensional image, for each predetermined category; and
train a plurality of the image generation models by individually training the image generation model for each category.

3. The image generation apparatus according to claim 2,
wherein the predetermined category is a category related to a person under examination.

4. The image generation apparatus according to claim 3,
wherein the category related to the person under examination includes at least one category of a height, a weight, a BMI, an age, mammary gland information, or a thickness of a breast of the person under examination.

5. The image generation apparatus according to claim 2,
wherein the predetermined category is a category related to a setting content during imaging.

6. The image generation apparatus according to claim 5,
wherein the category related to the setting content during the imaging includes at least one category of an irradiation angle of the radiation, a resolution of a captured image, or a dose mode of the radiation during the tomosynthesis imaging.

7. The image generation apparatus according to claim 2,
wherein the predetermined category is a category related to a type of an image processing technique for at least one of the plurality of projection images or the plurality of tomographic images or the normal two-dimensional image.

8. The image generation apparatus according to claim 7,
wherein the category related to the type of the image processing technique is a category for each manufacturer of an imaging apparatus that performs the tomosynthesis imaging.

9. The image generation apparatus according to any one of claims 2 to 8,
wherein the processor is configured to:
in a case in which an image quality of at least one image in the plurality of combinations of the plurality of projection images or the plurality of tomographic images and the normal two-dimensional image is lower than a predetermined level, prohibit use of the images of the combination for the training.

10. The image generation apparatus according to claim 2,
wherein the processor is configured to:
in a case in which the plurality of tomographic images are used for the training, correct the plurality of tomographic images to tomographic images in a case of virtually providing a tube at a position of the tube that emits the radiation in a case of capturing a corresponding normal two-dimensional image.

11. The image generation apparatus according to claim 2,
wherein the processor is configured to:
in a case in which a set of images, which is the combination of the plurality of projection images or the plurality of tomographic images and the normal two-dimensional image, is obtained by set imaging in which the tomosynthesis imaging and the capturing of the normal two-dimensional image are performed in the same compression state by the compression member, use the set of images as training data for the image generation model.

12. The image generation apparatus according to claim 2,
wherein the processor is configured to:
acquire the series of the plurality of projection images obtained by the tomosynthesis imaging in a state in which the breast is compressed by the compression member or the plurality of tomographic images obtained from the series of the plurality of projection images, as images for generation of the pseudo two-dimensional image; and
input the acquired images for generation to the image generation model to generate the pseudo two-dimensional image corresponding to the images for generation.

13. The image generation apparatus according to claim 12,
wherein the processor is configured to:
in a case in which the plurality of image generation models that have been individually trained for each predetermined category are provided as the image generation model, generate the pseudo two-dimensional image by selectively using the image generation model of the category corresponding to the pseudo two-dimensional image to be generated among the plurality of image generation models.

14. An image generation method executed by a computer, the image generation method comprising:
generating, via an image generation model (67), a pseudo two-dimensional image (102; 103) from a series of a plurality of projection images obtained by tomosynthesis imaging in a state in which a breast (U) is compressed by a compression member (30) or a plurality of tomographic images (100; 101) obtained from the series of the plurality of projection images,
wherein the image generation model has been trained in advance using a plurality of combinations of the plurality of projection images or the plurality of tomographic images and a normal two-dimensional image (111) captured by irradiating, with radiation (R), the breast in a state of being compressed by the compression member (30) during the tomosynthesis imaging for obtaining the plurality of projection images or the plurality of tomographic images.

15. A computer-readable storage medium storing a program that, when executing on a computer, causes the computer to execute a process comprising:
generating, via an image generation model (67), a pseudo two-dimensional image (102; 103) from a series of a plurality of projection images obtained by tomosynthesis imaging in a state in which a breast (U) is compressed by a compression member (30) or a plurality of tomographic images (100; 101) obtained from the series of the plurality of projection images,
wherein the image generation model has been trained in advance using a plurality of combinations of the plurality of projection images or the plurality of tomographic images and a normal two-dimensional image (111) captured by irradiating, with radiation (R), the breast in a state of being compressed by the compression member during the tomosynthesis imaging for obtaining the plurality of projection images or the plurality of tomographic images.
